# EUROPEAN PATENT SPECIFICATION

(11) **EP 4 106 715 B1**
(45) Date of publication and mention of the grant of the patent: **18.10.2023**
(21) Application number: 21702248.2
(22) Date of filing: 27.01.2021
(51) Int. Cl.: A61K 8/26, A61K 8/19, A61K 8/25, A61K 8/20, A61Q 11/00

(54) **HUMECTANT-FREE TOOTHPASTE COMPOSITION**
BEFEUCHTUNGSMITTELFREIE ZAHNPASTAZUSAMMENSETZUNG
COMPOSITION DE DENTIFRICE SANS HUMECTANT

(30) Priority: 18.02.2020 EP 20157958
(43) Date of publication of application: 28.12.2022
(73) Proprietor: Unilever IP Holdings B.V., 3013 AL Rotterdam (NL); Unilever Global IP Limited, Wirral, Merseyside CH62 4ZD (GB)
(72) Inventor: CHANDRASEKARAN, Sembian, Mumbai 400099 Andheri (IN); MORAKHIA, Mansi, Mukesh, Mumbai 400099 Andheri (IN)
(74) Representative: Tansley, Sally Elizabeth
(86) International application number: PCT/EP2021/051824
(87) International publication number: WO 2021/165001

(56) References cited:
- WO-A1-2014/147630
- WO-A1-2014/170096
- WO-A1-2015/094153
- WO-A1-2017/108368
- WO-A1-2019/104033
- US-A1- 2015 328 089

## Description

### Technical Field of the Invention

The present invention relates to toothpastes that contain very little humectant. More particularly the invention relates to toothpastes that do not contain humectants.

### Background of the Invention

Toothpaste compositions are widely used for oral care. Such compositions generally contain abrasives, humectants and thickeners. Humectants are present in almost all toothpastes and their amounts could vary from 10 wt% to 60 wt% of the composition. Some humectants inherently contain an appreciable amount of water. For example, commercial grade sorbitol contains 70% sorbitol and 30% water.

It is expected that a good toothpaste remains stable within a reasonably wide range of temperature. Sometimes toothpastes can experience fluctuations in temperature to which they are exposed. It is believed that 5°C to 45°C represents a range that is wide enough considering the temperature to which toothpastes are likely to be exposed. Therefore, when stability tests are performed in laboratories, the tests are often carried out within the aforementioned range. It is further believed that viscosity of chalk-based toothpastes should be in the range of 300 Pa. s ± 25 Pa. s (300000 cP ± 25000 cP) at 25°C.

Although humectants play an important role in toothpastes, they may interfere with certain rheological properties. In addition, humectants are expensive Therefore it is desirable to minimise their amount to the extent possible. In such cases, invariably, the compositions are thickened or stabilised by using one or more conventional thickening agents.

US2017135922A (P&G) discloses dentifrice composition comprising 45 to 75% water, 25 to 50% calcium-containing abrasive and 0.0025 to less than 7% basic amino acid in free or salt form (e.g., arginine bicarbonate (in acid or salt form). The composition comprises 0 to 10% humectant which can be sorbitol, glycerol or a combination thereof.

US2017333330 A (Colgate) discloses oral care compositions comprising sorbitol, and a thickening system having carrageenan and a combination of carboxymethylcellulose and xanthan. The oral care composition is free of glycerine but not sorbitol. The composition comprises water.

WO14170096 A1 (Unilever) discloses toothpastes having 20 to 60 wt% calcium-based abrasive, 3 to 15 wt% humectant and up to 2 wt% thickening silica. The composition further comprises 0.2 to 3 wt% smectite clay. Some compositions contain 0.8 wt% Veegum^{®} H which is a type of clay.

US10226410 B2 (P&G) discloses that humectants such as sorbitol and glycerol adversely affects the uptake of fluoride, therefore the chalk-based formulations disclosed in this patent do not contain humectant.

US2009269287 A1 (P&G) discloses thickened dentifrices in liquid, paste or gel form comprising a binding/thickening system that is also a humectant, thereby replacing a significant portion of traditional humectants like glycerine and sorbitol. The system comprises selected type of carrageenan that provides a water viscosity of at least about 20 mPa s and water-binding capacity to prevent loss of water.

WO17079952 A1 (P&G) discloses low-humectant or humectant-free toothpastes that are thickened with natural gums, e.g. gum karaya, gum arabic, gum tragacanth and xanthan gum.

US10130569 B2 (P&G, 2018) discloses aqueous dentifrice compositions containing a calcium-containing abrasive, alkaline pH and minimal polyols like PEG. Exemplified compositions contain SCMC and carrageenan.

WO14147630 A1 (Colgate) discloses toothpastes comprising a first dentifrice comprising a calcium carbonate abrasive and a second dentifrice comprising a zinc ion source in a gel base, wherein the second dentifrice is entrained as a stripe in the first dentifrice.

### Summary of the Invention

We have devised a new kind of thickening system that can stabilise toothpaste compositions which contain calcium-based abrasives. It is observed that the compositions maintain their viscosity within a defined range even within a wider temperature range. The toothpaste comprises a hydrophilic inorganic thickener which swells by intercalating water and an aluminium silicate clay other than the hydrophilic inorganic thickener. The toothpaste composition comprises minimum humectant, not more than 5 wt% in total.

In accordance with a first aspect is disclosed a toothpaste composition comprising:
(i) 20 to 60 wt% of one or more calcium-based abrasive;
(ii) 0.1 to 5 wt% hydrophilic inorganic thickener which swells by intercalating water;
(iii) 0.1 to 5 wt% aluminium silicate clay other than said hydrophilic inorganic thickener; and,
(iv) 0.05 to 2 wt% sodium chloride
   wherein sum total of humectants in said composition is not more than 5 wt% humectant.

All other aspects of the present invention will more readily become apparent upon considering the detailed description and examples which follow.

### Detailed Description

### Toothpaste

"Toothpaste" for the purpose of the present invention means a paste or gel dentifrice for use with a toothbrush.

### Refractive Index

Refractive index is quoted at a temperature of 25°C and a wavelength of 589 nm.

### pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

### Viscosity

Viscosity of a toothpaste is the value taken at room temperature (25 °C) with a Brookfield Viscometer, Spindle No.4 and at a speed of 5 rpm. Values are quoted in centipoises (cP=mPa.s) unless otherwise specified.

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy. Where a feature is disclosed with respect to a particular aspect of the invention (for example a composition of the invention), such disclosure is also to be considered to apply to any other aspect of the invention (for example a method of the invention) *mutatis mutandis.*

The present invention relates to a toothpaste composition comprising:
(i) 20 to 60 wt% calcium-based abrasive;
(ii) 0.1 to 5 wt% hydrophilic inorganic thickener which swells by intercalating water;
(iii) 0.1 to 5 wt% aluminium silicate clay other than said hydrophilic inorganic thickener which swells by intercalating water; and,
(iv) 0.05 to 2 wt% sodium chloride, wherein total amount of humectant in said composition is not more than 5 wt% humectant.

### Abrasive

Toothpaste compositions of the invention comprise 20 to 60 wt% calcium-based abrasive. The term calcium-based abrasive implies those abrasive materials that contain calcium. A variety of qualitative and quantitative methods are used to measure abrasive action of toothpastes. Quantitative methods include radioactive dentin abrasion (RDA) method, weight, and volume loss.

A variety of literature discloses information about abrasives that are used in toothpastes. An example is: Ferreira MC et.al., Effect of Toothpastes with Different Abrasives on Eroded Human Enamel: An in situ/ex vivo Study. Open Dent J. 2013;7:132-139. Published 2013 Sep 30. doi:10.2174/1874210601307010132

It is particularly preferred that the calcium-based abrasive is fine ground natural chalk (FGNC). FGNC may also be modified chemically or physically by coating during or after milling by heat treatment. Typical coating materials include magnesium stearate and oleate. The morphology of FGNC may also be modified during the milling process by using different milling techniques, for example, ball milling, air-classifier milling or spiral jet milling. FGNC can be used as the sole calcium containing abrasive. However, FGNC can also be used with other calcium containing abrasives to balance the abrasion.

Alternatively, the calcium-based abrasive is dicalcium phosphate (DCP), calcium pyrophosphate or precipitated calcium carbonate (PCC). It is further preferred that the toothpaste composition of the invention comprises a combination of calcium-based abrasives. In such a case it is preferred that FGNC accounts for 35 to 100 % of the total amount of calcium-based abrasives, more preferably 75 to 100 % and especially from 95 to 100 %. In such cases, the balance, most preferably, is PCC.

The toothpaste composition of the invention may optionally comprise other abrasives depending upon the intended degree of abrasion. These include synthetic abrasive polishing agents such as magnesium carbonate, sodium metaphosphate, potassium metaphosphate, zirconium silicate, potassium metaphosphate, magnesium orthophosphate, magnesium orthophosphate, trimagnesium phosphate, aluminum silicate, zirconium silicate and perlite.

### Humectant

The sum total of humectants in the composition is not more than 5 wt%. It is preferred that the humectant is selected from the group consisting of glycerin, sorbitol, xylitol, butylene glycol and propylene glycol. Further preferably the sum total is not more than 2 wt%. Yet further preferably the composition does not comprise any humectant selected from the group consisting of glycerin, sorbitol, xylitol, butylene glycol and propylene glycol.

Humectants retain moisture so that the dentifrice does not dry out. Humectants bind and hold the solvent in the dentifrice. Water is the solvent used in most dentifrices. Humectants, such as glycerin and sorbitol, also provide flowability to the dentifrice.

### Inorganic thickener

It is preferred that the hydrophilic inorganic thickener which swells by intercalating water is at least one of magnesium-aluminium silicate, sodium magnesium silica, a layered silicate, smectite, hectorite, bentonite, montmorillonite or phyllosilicate.

A particularly preferred thickener is a 2:1 layered clay, more preferably of smectite group. A preferred magnesium aluminium silicate is commercially available as VEEGUM^{®} from R. T. Vanderbilt Company. A preferred sodium magnesium silicate is commercially available as LAPONITE^{®}. Other preferred organically modified smectites including tetra alkyl and/or trialkyl ammonium smectites (organically modified montmorillonite clays) such as quaternium-18 bentonite, quaternium-18 hectorite, stearalkonium bentonite and stearalkonium hectorite/ and mixtures thereof.

### Aluminium silicate clay

It is preferred that the aluminium silicate clay is a 1:1 layered silicate. More preferably it is kaolin. Kaolinites have a dioctahedral sheet and the species included within the kaolinites subgroup are kaolinite, dickite, nacrite and halloysite clay minerals, but not limited thereto.

Kaolin (also known as kaolinite) is a naturally occurring mineral. Preferred kaolin is a hydrous aluminium silicate and represented as Al₂O₃•2SiO₂•2H₂O. Preferred kaolinites have particle size distribution such that at least 98 wt% of the particles have a particle size of 2 µm or less.

It is particularly preferred that the composition comprises an amount of said aluminium silicate clay and an amount of said hydrophilic inorganic thickener such that ratio of the amount of said clay to that of said thickener is from 1:1 to 1:4 parts by weight.

### Surfactant

It is preferred that the toothpaste composition comprises a surfactant. Preferably the composition comprises at least 0.01 wt% surfactant, more preferably at least 0.1 wt%, and most preferably from 0.5 to 7 wt%. Suitable surfactants include anionic surfactants, such as the sodium, magnesium, ammonium or ethanolamine salts of C8 to C18 alkyl sulphates (for example sodium lauryl sulphate), C8 to C16 alkyl sulphosuccinates (for example dioctyl sodium sulphosuccinate), C8 to C12 alkyl sulphoacetates (such as sodium lauryl sulphoacetate), C8 to C24 alkyl sarcosinates (such as sodium lauryl sarcosinate), C8 to C24 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate, sodium coco sulfate, cocamidopropyl betaine or mixtures thereof.

### Other thickener

It is preferred that the toothpaste compositions of the invention comprise 0.1 to 8 wt% of a thickener other than the inorganic thickener.

It is preferred that the toothpaste composition of the invention comprises 0.1 to 3 wt%, more preferably 0.1 to 2 wt% thickener. It is preferred that the thickener is a cellulosic thickener selected from sodium carboxymethyl cellulose (SCMC), hydroxyl ethyl cellulose, methyl cellulose or ethyl cellulose.

Alternatively, the thickener is thickening silica. It is preferred that their refractive index is in the range of 1.445 to 1.460.

Further preferably the toothpaste composition of the invention comprises a cellulosic thickener and thickening silica. In such cases it is preferred that the composition comprises 0.5 wt% to 6 wt% thickening silica and 0.1 to 1 wt% cellulosic thickener. Preferably the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4.

The compositions of the invention may further comprise one or more thickeners selected from gum tragacanth, gum arabic, gum karaya, sodium alginate, carrageenan, guar, xanthan gum, Irish moss, starch, modified starch, Carbomers (cross-linked acrylates) and mixtures thereof. When a Carbomer is employed, those having a weight-average molecular weight of at least 700,000 Da are desired, and preferably, those having a molecular weight of at least 1,200,000 Da and most preferably, those having a molecular weight of at least about 2,500,000 Da are desired. Mixtures of Carbomers may also be used herein.

### Viscosity

It is preferred that viscosity of chalk-based toothpaste composition of the invention is in the range of 250 Pa. s to 350 Pa. s (250000 cP to 350000 cP) at 25°C. More preferably the viscosity is from 280 Pa. s to 320 Pa.s (280,000 cP to 320,000 cP.). Viscosity of toothpastes can fluctuate within a wide-range and it primarily depends on the temperature experienced by the composition. Therefore, in order to test the robustness of such compositions, formulation scientists usually conduct storage stability tests at temperatures ranging from 0°C to 45°C. Such tests are generally carried out for a duration of at least one month and it could also extend to three months. Observations are recorded periodically and at least at initial stage and then at the end of the test period. Then depending on the initial and final observations, the scientists determine the extent of stability of the tested compositions

### Other benefit agents

The toothpaste composition may further comprise benefit agents that are typically delivered to human teeth and/or the oral cavity including the gums to enhance or improve a characteristic of those dental tissues. The only limitation with respect to the benefit agents that may be used in this invention is that the same is suitable for use in the mouth. The benefit agents are present in the oral care composition in addition to the composite material that is included in the composition.

Typically the benefit agent is selected from optical agents, biomineralization agents, antibacterial agents, gum health agents, desensitizing agents, anti-calculus agents, freshness agents or mixtures thereof. Preferably, the benefit agent is selected from optical agents, antibacterial agents, gum health agents, freshness agents or mixtures thereof.

For example, optical agents such as colouring agents like whitening agents and pigments. Preferably, the pigment, when used, is violet or blue having a hue angle, h, in the CIELAB system of from 220 to 320 degrees. These pigments may be selected from one or more of those listed in the Colour Index International, listed as pigment blue 1 through to pigment blue 83, and pigment violet 1 through to pigment violet 56. In another preferred embodiment, the optical agents may be selected from one or more of mica, interference mica, boron nitride, poly(methyl methacrylate) flake, composite microspheres, titanium dioxide coated glass flake, inverse opal, cholesteric liquid crystal, photonic sphere, hollow sphere and zinc oxide. Antibacterial agents may be selected from one or more of metal salts where the metal is selected from zinc, copper, silver or a mixture thereof, triclosan, triclosan monophosphate, triclocarban, curcumin, quaternary ammonium compounds, bisbiguanides and long chain tertiary amines, preferably zinc salts including zinc oxide, zinc chloride, zinc acetate, zinc ascorbate, zinc sulphate, zinc nitrate, zinc citrate, zinc lactate, zinc peroxide, zinc fluoride, zinc ammonium sulfate, zinc bromide, zinc iodide, zinc gluconate, zinc tartarate, zinc succinate, zinc formate, zinc phenol sulfonate, zinc salicylate, zinc glycerophosphate or a mixture thereof. Gum health agents may be selected from one or more of anti-inflammatory agents, plaque buffers, biomolecules, proteinaceous materials, vitamin, plant extracts and curcumin. Freshness agents may be flavors selected from one or more of peppermint, spearmint, menthol, flora oil, clove oil and citrus oil.

In a preferred embodiment, the benefit agent is a particulate whitening agent for tooth whitening. Typically, the particulate whitening agent comprises a material suitable to physically and immediately improve characteristics of teeth and especially whiten teeth. In order to provide excellent whitening effect it preferred that refractive index is least 1.9, more preferably at least 2.0, even more preferably at least 2.2, even more preferably still at least 2.4 and most preferably at least 2.5. The maximum refractive index of the material is not particularly limited but preferably up to 4.0. Preferably, the refractive index is from 1.9 to 4.0.

In a preferred embodiment, the particulate whitening agent comprises metal oxides, nonmetal oxides or a combination thereof in an amount of at least 50% by weight of the whitening agent and more preferably at least 70%, more preferably still from 80 to 100% and most preferably from 85 to 95%. In an especially preferred embodiment, the particulate whitening agent is at least 50% by weight titanium dioxide, and most preferably, from 60 to 100% by weight titanium dioxide, based on total weight of the whitening agent and including all ranges subsumed therein. In another especially preferred embodiment, the particulate whitening agents are slightly soluble or insoluble in water, but most preferably, insoluble in water.

In an especially preferred embodiment, the particulate whitening agent is titanium dioxide coated with calcium silicate. The particulate whitening agent according to the present invention can be of different sizes and shapes. The particles may be of spherical, platelet or irregular shape form. The diameter of the particulate whitening agent is often from 10 nm to less than 50 microns, and preferably, from 75 nm to less than 10 microns. In an especially preferred embodiment, the diameter of particles is from 100 nm to 5 microns, including all ranges subsumed therein. Particle size can be measured, for example, by dynamic light scattering (DLS). For composite particles, in a preferred embodiment, at least 40%, and preferably, at least 60%, and most preferably, from 75 to 99.5% of the diameter of the composite particle is the core, including all ranges subsumed therein.

The toothpaste composition of the present invention may comprise a single benefit agent or a mixture of two or more benefit agents. Typically, the benefit agent is present in an amount from 0.25 to 10%, and more preferably, from 0.5 to 8%, and most preferably, from 1 to 30% by total weight of the oral care composition and including all ranges subsumed therein.

The compositions of this invention are prepared by conventional methods of making oral care formulations. Such methods include mixing the ingredients under moderate shear and atmospheric pressure. The compositions are used in the oral cavity, and preferably, are of the form that may be brushed onto teeth with a toothbrush.

Typically, the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area.

The composition can be effective even when used in an individual's daily oral hygiene routine. For example, the composition may be brushed onto the teeth. The composition may be used daily, for example for use by an individual once, twice or three times per day.

The following examples are provided to facilitate an understanding of the present invention. The examples are not provided to limit the scope of the claims.

### Examples

### Example 1

Five humectant-free toothpastes were prepared by following standard procedure. Only one composition (No. 1) was within the invention. Details are shown in Table 1.

**Table 1**

| **Ingredient** | **Reference number/wt% of the ingredient** | | | | |
|---|---|---|---|---|---|
| | **A** | **B** | **C** | **D** | **1** |
| Water | 45.0 | 45.0 | 46.0 | 45.0 | 45.0 |
| FGNC 1005 | 40.0 | 40.0 | 40.0 | 40.0 | 40.0 |
| MFIL thickening silica | 5.0 | 5.0 | 5.0 | 5.0 | 5.0 |
| SLS | 2.5 | 2.5 | 2.5 | 2.5 | 2.5 |
| Kaolin | -- | 1.0 | -- | 1.0 | 1.0 |
| Veegum^{®} Ultra | -- | -- | 0.5 | 0.5 | 0.5 |
| Sodium chloride | -- | -- | -- | -- | 0.2 |
| SCMC - 9H | 0.7 | 0.7 | 0.7 | 0.7 | 0.7 |
| Other minors to | 100.0 | 100.0 | 100.0 | 100.0 | 100.0 |

All the compositions were subjected to a test to find out which ones were stable across of range of temperatures that are commonly encountered during manufacture, transit and storage of such toothpastes. For that purpose, randomised samples of each toothpaste were packaged in collapsible tubes. Then the tubes were stored for one month under conditions indicated in the table 2. Viscosity was determined at the end of the month. In the table 2, initial means measured after preparing the composition.

**Table 2**

| **Temperature of storage for 1 month** | **Viscosity in '000/cP** | | | | |
|---|---|---|---|---|---|
| | **A** | **B** | **C** | **D** | 1 |
| Initial at 25°C | 105 | 127 | 271 | 226 | 310 |
| 5°C | 130 | 122 | 360 | 300 | 300 |
| 25°C | 132 | 123 | 375 | 302 | 304 |
| 40°C | 124 | 120 | 400 | 350 | 320 |
| 45°C | 133 | 104 | 400 | 400 | 318 |

Data in table 2 indicates that viscosity of composition of the present invention (1) does not alter drastically (when compared with the corresponding initial) when stored at 5°C for a month or even at 45°C for a month. This indicates that the composition of the invention is the most stable. Viscosity of the compositions A and B were not as high as expected, whereas in the case of C and D, the compositions progressively turned viscous as the temperature increased, thereby indicating that C and D remained robust or stable enough.

### Example 2: Data and observations about a marketed product (*MP)

In another series of experiments, a marketed toothpaste comprising apprx. 50 wt% water, 40 wt% calcium carbonate, 0.3 wt% carrageenan and 0.4 wt% SCMC was subjected to weight-loss study to determine whether water present in the composition was retained over a period and to what extent. In other words, the % weight loss was recorded (n=3) at frequency of 2 hours for a total of 8 hours on the first day (day-1) of the study and then repeated on day-2. This experiment was conducted at 25°C under standard operating procedure. All the compositions of Table 1 were also subjected to the same test.

The data is presented in Table 3.

**Table 3**

| **Reference number** | **% weight loss/day 1 at the end of** | | | | **% weight loss/day 2 at the end of** | | | | |
|---|---|---|---|---|---|---|---|---|---|
| | **2 hr** | **4 hr** | **6 hr** | **8 hr** | **T=0** | **2 hr** | **4 hr** | **6 hr** | **8 hr** |
| A | 6.8 | 13.2 | 18.9 | 25.3 | 41.0 | 41.0 | 43.1 | 43.8 | 44.2 |
| B | 7.3 | 13.6 | 18.9 | 24.7 | 41.1 | 43.4 | 44.2 | 44.7 | 44.9 |
| C | 6.4 | 11.6 | 16.6 | 21.8 | 39.0 | 41.8 | 43.2 | 44.0 | 44.4 |
| D | 6.5 | 12.0 | 17.0 | 22.5 | 39.1 | 41.6 | 42.5 | 43.4 | 43.5 |
| 1 | 6.9 | 12.2 | 17.0 | 23.0 | 38.0 | 40.2 | 41.3 | 42.2 | 42.4 |
| *MP | 7.5 | 13.4 | 19.0 | 25.5 | 46.7 | 50.6 | 52.5 | 54.1 | 54.5 |

The data indicates that the marketed product containing carrageenan and SCMC lost maximum weight which is an indication of significant loss of moisture. In such cases, it would be necessary to package the toothpaste in tubes having extremely good moistureretaining property. On the other hand, the observations concerning compositions A-D and 1 were similar. However, the technical benefits of composition 1 which is as per the present invention has been demonstrated in Example 1.

## Claims

1. A toothpaste composition comprising:
(i) 20 to 60 wt% of one or more calcium-based abrasive;
(ii) 0.1 to 5 wt% hydrophilic inorganic thickener which swells by intercalating water;
(iii) 0.1 to 5 wt% aluminium silicate clay other than said hydrophilic inorganic thickener; and,
(iv) 0.05 to 2 wt% sodium chloride
wherein sum total of humectants in said composition is not more than 5 wt% humectant, wherein said hydrophilic inorganic thickener is at least one of magnesium-aluminium silicate, sodium magnesium silica, a layered silicate, smectite, hectorite, bentonite, montmorillonite or a phyllosilicate.

2. A toothpaste composition as claimed in claim 1 wherein said composition comprises an amount of said aluminium silicate clay and an amount of said hydrophilic inorganic thickener such that ratio of the amount of said clay to that of said thickener is from 1:1 to 1:4 parts by weight.

3. A toothpaste composition as claimed in any of claims 1 or 2 wherein said aluminium silicate clay is a 1:1 layered silicate.

4. A toothpaste composition as claimed in claim 3 wherein said clay is kaolin.

5. A toothpaste composition as claimed in any of claims 1 to 5 wherein said humectant is selected from the group consisting of glycerin, sorbitol, xylitol, butylene glycol and propylene glycol.

6. A toothpaste composition as claimed in any of claims 1 to 5 wherein sum total of humectants in said composition is not more than 2 wt%.

7. A toothpaste composition as claimed in claim 6 wherein said composition does not comprise any humectant selected from the group consisting of glycerin, sorbitol, xylitol, butylene glycol and propylene glycol.

8. A toothpaste composition as claimed in any of claims 1 to 7 comprising 0.1 to 8 wt% of a thickener.

9. A toothpaste composition as claimed in claim 8 wherein said thickener is thickening silica having refractive index in the range of 1.445 to 1.460.

10. A toothpaste composition as claimed in claim 9 wherein said composition comprises a cellulosic thickener and thickening silica.

11. A toothpaste composition as claimed in any of claims 1 to 10 wherein viscosity of composition is in the range of 250 Pa. s (250000 cP) to 350 Pa.s (350000 cP) at 25°C, where said viscosity is taken at room temperature (25 °C) with a Brookfield Viscometer, Spindle No.4 and at a speed of 5 rpm.

## Patentansprüche

1. Zahnpastazusammensetzung, umfassend:
(i) 20 bis 60 Gew.-% eines oder mehrerer Schleifmittel auf Calciumbasis;
(ii) 0,1 bis 5 Gew.-% hydrophiles anorganisches Verdickungsmittel, das durch Einlagerung von Wasser quillt;
(iii) 0,1 bis 5 Gew.-% Aluminiumsilikatton, außer dem hydrophilen anorganischen Verdickungsmittel; und
(iv) 0,05 bis 2 Gew.-% Natriumchlorid,
wobei die Gesamtsumme der Feuchthaltemittel in der Zusammensetzung nicht mehr als 5 Gew.-% Feuchthaltemittel beträgt, wobei das hydrophile anorganische Verdickungsmittel mindestens eines von Magnesium-Aluminium-Silikat, Natrium-Magnesium-Siliziumdioxid, einem Schichtsilikat, Smektit, Hektorit, Bentonit, Montmorillonit oder einem Phyllosilikat ist.

2. Zahnpastazusammensetzung, wie im Anspruch 1 beansprucht, wobei die Zusammensetzung eine solche Menge des Aluminiumsilikattons und eine solche Menge des hydrophilen anorganischen Verdickungsmittels umfasst, dass das Verhältnis der Menge des Tons zu der des Verdickungsmittels 1:1 bis 1:4 Gewichtsteile beträgt.

3. Zahnpastazusammensetzung, wie in einem der Ansprüche 1 oder 2 beansprucht, wobei der Aluminiumsilikatton ein 1:1-Schichtsilikat ist.

4. Zahnpastazusammensetzung, wie im Anspruch 3 beansprucht, wobei der Ton Kaolin ist.

5. Zahnpastazusammensetzung, wie in einem der Ansprüche 1 bis 5 beansprucht, wobei das Feuchthaltemittel aus der Gruppe ausgewählt ist, bestehend aus Glycerin, Sorbit, Xylit, Butylglycol und Propylenglycol.

6. Zahnpastazusammensetzung, wie in einem der Ansprüche 1 bis 5 beansprucht, wobei die Gesamtsumme des Feuchthaltemittels in der Zusammensetzung nicht mehr als 2 Gew.-% beträgt.

7. Zahnpastazusammensetzung, wie im Anspruch 6 beansprucht, wobei die Zusammensetzung kein Feuchthaltemittel enthält, ausgewählt aus der Gruppe, bestehend aus Glycerin, Sorbit, Xylit, Butylglycol und Propylenglycol.

8. Zahnpastazusammensetzung, wie in einem der Ansprüche 1 bis 7 beansprucht, umfassend 0,1 bis 8 Gew.-% eines Verdickungsmittels.

9. Zahnpastazusammensetzung, wie im Anspruch 8 beansprucht, wobei das Verdickungsmittel verdickendes Siliziumdioxid mit einem Brechungsindex im Bereich von 1,445 bis 1,460 ist.

10. Zahnpastazusammensetzung, wie im Anspruch 9 beansprucht, wobei die Zusammensetzung ein Celluloseverdickungsmittel und verdickendes Siliziumdioxid umfasst.

11. Zahnpastazusammensetzung, wie in einem der Ansprüche 1 bis 10 beansprucht, wobei die Viskosität der Zusammensetzung bei 25°C in dem Bereich von 250 Pa.s (250000 cP) bis 350 Pa.s (350000 cP) ist, wobei die Viskosität bei Raumtemperatur (25°C) mit einem Brookfield-Viscometer, Spindel-Nr. 4 und bei einer Geschwindigkeit vom 5 U/min gemessen wird.

## Revendications

1. Composition de pâte dentifrice comprenant :
(i) 20 à 60 % en poids d'un ou plusieurs abrasifs à base de calcium ;
(ii) 0,1 à 5 % en poids d'un épaississant inorganique hydrophile qui gonfle par intercalation d'eau ;
(iii) 0,1 à 5 % en poids d'argile de type silicate d'aluminium autre que ledit épaississant inorganique hydrophile ; et
(iv) 0,05 à 2 % en poids de chlorure de sodium
dans laquelle la somme totale des humectants dans ladite composition n'est pas supérieure à 5 % en poids d'humectant, dans laquelle ledit épaississant inorganique hydrophile est au moins l'un parmi le silicate de magnésium et d'aluminium, la silice sodique magnésique, un silicate lamellaire, la smectite, l'hectorite, la bentonite, la montmorillonite, et un phyllosilicate.

2. Composition de pâte dentifrice selon la revendication 1, laquelle composition comprend une quantité de ladite argile de type silicate d'aluminium et une quantité dudit épaississant inorganique hydrophile telles que le rapport de la quantité de ladite argile à celle dudit épaississant soit de 1/1 à 1/4 parties en poids.

3. Composition de pâte dentifrice selon l'une quelconque des revendications 1 et 2, dans laquelle ladite argile de type silicate d'aluminium est un silicate lamellaire 1/1.

4. Composition de pâte dentifrice selon la revendication 3, dans laquelle ladite argile est le kaolin.

5. Composition de pâte dentifrice selon l'une quelconque des revendications 1 à 5, dans laquelle ledit humectant est choisi dans le groupe constitué par la glycérine, le sorbitol, le xylitol, le butylèneglycol et le propylèneglycol.

6. Composition de pâte dentifrice selon l'une quelconque des revendications 1 à 5, dans laquelle la somme totale des humectants dans ladite composition n'est pas supérieure à 2 % en poids.

7. Composition de pâte dentifrice selon la revendication 6, laquelle composition ne comprend aucun humectant choisi dans le groupe constitué par la glycérine, le sorbitol, le xylitol, le butylèneglycol et le propylèneglycol.

8. Composition de pâte dentifrice selon l'une quelconque des revendications 1 à 7, comprenant 0,1 à 8 % en poids d'un épaississant.

9. Composition de pâte dentifrice selon la revendication 8, dans laquelle ledit épaississant est une silice épaississante ayant un indice de réfraction situé dans la plage allant de 1,445 à 1,460.

10. Composition de pâte dentifrice selon la revendication 9, laquelle composition comprend un épaississant cellulosique et une silice épaississante.

11. Composition de pâte dentifrice selon l'une quelconque des revendications 1 à 10, dans laquelle la viscosité de la composition est située dans la plage allant de 250 Pa.s (250 000 cP) à 350 Pa.s (350 000 cP) à 25°C, où ladite viscosité est prise à la température ambiante (25°C) avec un viscosimètre Brookfield, broche N° 4 et à une vitesse de 5 t/min.
